# FASCICULE DE BREVET EUROPEEN

(11) **EP 1 303 224 B1**
(45) Date de publication et mention de la délivrance du brevet: **08.06.2005**
(21) Numéro de dépôt: 01958175.0
(22) Date de dépôt: 25.07.2001
(51) Int. Cl.: A61B 17/70

(54) **PIECE DE LIAISON SEMI-RIGIDE POUR LA STABILISATION DU RACHIS**
HALBSTEIFES VERBINDUNGSSTÜCK ZUR STABILISIERUNG DER WIRBELSÄULE
SEMIRIGID LINKING PIECE FOR STABILISING THE SPINE

(30) Priorité: 25.07.2000 FR 0009705
(43) Date de publication de la demande: 23.04.2003
(73) Titulaire: Spine Next, La Cité Mondiale, 33000 Bordeaux (FR)
(72) Inventeur: LE COUEDIC, Régis, F-33000 BORDEAUX (FR); PASQUET, Denis, F-PESSAC 33600 (FR)
(74) Mandataire: Dronne, Guy
(86) Numéro de dépôt international: PCT/FR2001/002425
(87) Numéro de publication internationale: WO 2002/007621

(56) Documents cités:
- EP-A- 0 667 127
- FR-A- 2 755 844
- US-A- 5 415 661

## Description

La présente invention concerne une pièce de liaison destinée à maintenir un espacement entre au moins deux éléments d'ancrage reliés par ladite pièce de liaison.

Des domaines d'application de l'invention sont notamment la stabilisation et les arthrodèses de segments de la colonne vertébrale dans les pathologies dégénératives du rachis.

Des systèmes de stabilisation de la colonne vertébrale maintenant au moins deux vertèbres consécutives au moyen d'éléments d'ancrages fixés dans lesdites vertèbres et reliés par des tiges de liaison rigides, sont bien connus. De tels systèmes sont généralement accouplés de façon à relier deux vertèbres consécutives par deux tiges sensiblement parallèles fixées de chaque côté des apophyses épineuses. Les éléments d'ancrage vissés dans la partie postérieure des vertèbres traversent les pédicules et une partie substantielle des corps vertébraux de façon à obtenir une liaison fixe et durable dans le temps.

Ces systèmes de stabilisation sont couramment utilisés pour consolider plusieurs vertèbres consécutives. Ainsi, les vertèbres sont-elles reliées les unes aux autres au moyen de tiges rigides sur une longueur substantielle de la colonne vertébrale. De tels montages permettent d'assurer correctement le maintien des vertèbres les unes par rapport aux autres ; en revanche, ils raidissent considérablement la flexion du rachis. Il a été démontré qu'un système de stabilisation plus flexible, conférant aux vertèbres une plus grande mobilité relative entre elles, était bénéfique dans le cas de certaines pathologies.

Tout naturellement, pour augmenter l'amplitude de flexion du système de stabilisation il a été proposé de diminuer la section des tiges de liaison entre les éléments d'ancrage afin d'augmenter la déformation desdites tiges de liaison pour une même contrainte. Ainsi, la mobilité relative des vertèbres, les unes par rapport aux autres, est plus importante et l'amplitude de flexion avant/arrière de la colonne vertébrale est plus grande pour un même effort. Cependant, la stabilisation de la colonne vertébrale est moins bien assurée, en particulier la stabilisation latérale des vertèbres les unes par rapport aux autres. Par ailleurs, les contraintes s'exerçant sur les tiges de liaison dont la section est moindre sont plus importantes de sorte que les tiges de liaison sont susceptibles de se détériorer prématurément. FR-A-2 755 844 divulgue un autre système d'ostéosynthèse pour colonne vertébrale dont l'organe de liaison (6) présente deux branches dont l'une comporte une échancrure (36) et n'est par consequent pas continue.

Un premier objet de la présente invention est de fournir une pièce de liaison apte à maintenir espacés des éléments d'ancrage existants, ladite pièce de liaison présentant une amplitude de flexion accrue par rapport aux tiges de liaison actuellement utilisées sans pour autant augmenter les contraintes internes subies par lesdites tiges de liaison.

Pour atteindre ce but, conformément à l'invention, la pièce de liaison destinée à maintenir un espacement entre au moins deux éléments d'ancrage vissés dans des vertèbres comprend au moins : une partie flexible divisée en deux branches continues espacées l'une de l'autre, lesdites branches étant, sensiblement symétriques par rapport à l'axe longitudinal de ladite pièce, les extrémités desdites branches étant reliées entre elles deux à deux et définissant un premier plan moyen, et, deux parties rigides formant tiges, présentant une première portion de fixation et une deuxième portion, chaque dite deuxième portion desdites deux parties rigides prolongeant respectivement dans des directions opposées lesdites extrémités desdites branches reliées entre elles deux à deux, la section droite de chacune desdites branches étant inférieure à la section droite desdites parties rigides de façon que ladite pièce de liaison, dont lesdites portions de fixation sont fixées respectivement sur chacun des deux éléments d'ancrage, soit apte à fléchir élastiquement autour d'un axe perpendiculaire à l'axe longitudinal, ledit axe perpendiculaire étant contenu dans ledit plan moyen de la pièce de liaison lors du déplacement relatif des vertèbres, par quoi les vertèbres, maintenues espacées l'une de l'autre, sont mobiles l'une par rapport à l'autre.

Ainsi, une caractéristique de la pièce de liaison réside dans la forme de la pièce sur les parties rigides de laquelle les contraintes exercées par la flexion du rachis entraînent le fléchissement des deux branches perpendiculairement au plan moyen qu'elles forment. En effet, le fléchissement de la pièce de liaison n'est possible que selon la perpendiculaire au plan moyen que forment les deux branches, car elles sont réunies à chacune de leurs extrémités par les parties rigides et le fléchissement selon un autre axe entraînerait l'élongation d'une des branches et la compression longitudinale de l'autre ; ce qui, compte tenu des contraintes, entraînerait de faibles déformations. De la sorte, la pièce de liaison comporte une direction déterminée de fléchissement perpendiculaire à son axe principal et elle est disposée de façon que le plan moyen, défini par les deux branches, soit sensiblement perpendiculaire au plan dans lequel le rachis est apte à fléchir. Comme on l'expliquera plus en détails, la section droite de chacune des branches continues qui est sensiblement constante, est inférieure à la section droite des parties rigides, de sorte que l'amplitude de flexion de la pièce est plus importante pour un même effort. En outre, les contraintes à la flexion, représentatives des forces internes que subissent les deux branches, sont inférieures aux contraintes que subirait une seule branche présentant le même fléchissement sous le même effort. Ainsi, la fatigue de la pièce de liaison selon l'invention est diminuée.

Avantageusement, la pièce de liaison comprend n parties rigides entre lesquelles sont interposées n-1 parties flexibles selon l'axe longitudinal de ladite pièce, chaque partie rigide située entre deux parties flexibles comportant une première portion de fixation et deux deuxièmes portions, chacune desdites deuxièmes portions étant située de chaque côté de ladite première portion de fixation, lesdites deuxièmes portions prolongeant respectivement les extrémités des branches desdites deux parties flexibles de façon que les plans moyens de toutes les parties flexibles soient sensiblement situés dans un même plan, et en ce que les parties rigides situées aux deux extrémités de ladite pièce présente une seule deuxième portion prolongeant les extrémités des branches des parties flexibles, par quoi ladite pièce est apte à relier n éléments d'ancrage.

Ainsi, selon cette caractéristique, la pièce de liaison maintient un espacement entre tous les éléments d'ancrage qu'elle relie, ces derniers étant susceptibles d'être fixés chacun dans une vertèbre, de façon à constituer un alignement. Chaque partie rigide est fixée à un élément d'ancrage et entre chaque élément d'ancrage une partie flexible est prolongée par lesdites parties rigides. De la sorte, une pluralité de vertèbres est stabilisée avec une seule pièce de liaison unique, ce qui permet de diminuer le temps de montage de l'ensemble du système de stabilisation et par conséquent le temps opératoire. En outre, cette caractéristique de la pièce de liaison permet d'assurer une stabilisation sur plusieurs vertèbres consécutives en les reliant entre elles, tout en leur permettant une flexibilité relative importante.

Selon un mode particulier de réalisation de ladite pièce de liaison, la somme de la surface des sections desdites deux branches est inférieure à la surface de la section desdites parties rigides formant tiges. Ainsi, les parties rigides formant tiges sont rigides au regard des deux branches qui fléchissent préférentiellement, lesdites parties rigides étant solidement reliées auxdits éléments d'ancrage.

Selon un autre mode particulier de réalisation de l'invention, lesdites branches présentent des portions parallèles entre elles et à l'axe longitudinal de ladite pièce et la distance qui sépare lesdites branches est au moins égale à la longueur desdites portions. Cette configuration permet d'obtenir une pièce de liaison dont le fléchissement selon une direction perpendiculaire au plan moyen défini par les branches est très important par rapport au fléchissement selon une autre direction ; la pièce de liaison présente ainsi une seule direction de fléchissement compte tenu des efforts qui s'exercent sur elle.

Selon encore un autre mode particulier de réalisation de l'invention, la distance qui sépare les extrémités desdites branches reliées deux à deux est comprise entre 1,5 et 2,5 fois la distance qui sépare lesdites branches. Cette particularité permet d'obtenir un fléchissement supérieur au fléchissement des tiges de liaison couramment utilisées tout en procurant une seule direction de fléchissement perpendiculaire au plan moyen défini par les deux branches.

Par ailleurs, de façon avantageuse, la section desdites parties rigides formant tiges est circulaire, ce qui facilite la réalisation de la pièce qui, préférentiellement, est en alliage de titane. Par ailleurs, dans le cas où des tiges de liaison de section circulaire conformes à celles de l'art antérieur sont remplacées par des pièces de liaison conformes à l'invention sans avoir besoin de remplacer les éléments d'ancrage, il est nécessaire que lesdites parties rigides comportent des sections identiques aux sections des tiges de liaison de l'art antérieur.

Les alliages de titane présentent des propriétés mécaniques et antioxydantes compatibles avec les caractéristiques techniques recherchées pour la pièce de liaison.

Un deuxième objet de la présente invention est de proposer un système de stabilisation vertébrale destiné à solidariser au moins deux vertèbres, lesdites vertèbres présentant chacune un plan moyen sensiblement perpendiculaire à l'axe du rachis qu'elles forment et une paroi postérieure définissant un plan moyen postérieur dudit rachis, ledit système comportant au moins deux éléments d'ancrage aptes à être fixés chacun dans la paroi postérieure d'une vertèbre de façon que la ligne qui coupe lesdits deux éléments d'ancrage soit sensiblement parallèle audit axe du rachis, ledit système comprenant au moins une pièce de liaison selon l'invention, apte à relier lesdits deux éléments d'ancrage par lesdites deux parties rigides de façon que ledit plan moyen défini par lesdites deux branches soit sensiblement parallèle audit plan moyen postérieur dudit rachis, par quoi lesdites vertèbres, reliées dans leur partie postérieure, présentent une mobilité relative selon ledit axe dudit rachis.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 est une vue schématique en perspective de la pièce de liaison conforme à l'invention,
- la Figure 2, est une vue schématique en perspective montrant les éléments d'ancrage reliés par la pièce de liaison, et,
- la Figure 3 est une vue schématique latérale en élévation de la colonne vertébrale montrant deux vertèbres consécutives dans lesquelles sont vissés les éléments d'ancrage , lesdits éléments d'ancrage étant reliés par la pièce de liaison conforme à l'invention.

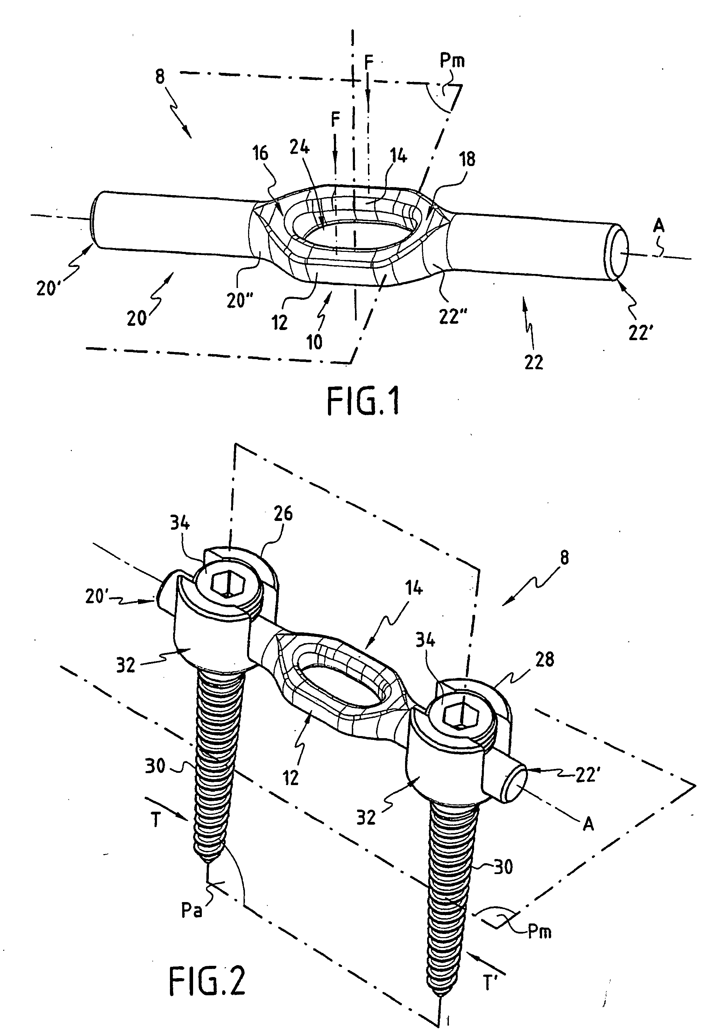

En se référant tout d'abord à la Figure 1 on décrira les différentes parties qui constituent une pièce de liaison selon l'invention.

La pièce de liaison 8 comporte une partie flexible 10 présentant deux branches 12 et 14, parallèles entre elles et réunies dans leurs extrémités en deux points 16 et 18. Deux parties rigides 20 et 22 formant tiges, présentant une première portion de fixation 20', 22' et une deuxième portion 20", 22" prolongeant les extrémités réunies des deux branches 12 et 14 à partir des deux points 16 et 18 dans des directions opposées selon un axe longitudinal A. Ainsi, les deux branches 12 et 14, parallèles entre elles et réunies dans leurs extrémités entourent un évidement 24. Par ailleurs elles sont parallèles à l'axe A et symétriques par rapport à ce dernier. De préférence, la somme des sections droites des deux branches 12 et 14 est inférieure à la section des parties rigides qui présentent sensiblement la même section circulaire, de sorte que le diamètre de la section droite d'une branche est inférieur à 70% du diamètre des parties rigides. Par exemple, le diamètre de la section droite d'une branche est compris entre 50 et 70% du diamètre des parties rigides.

En outre, les deux branches 12 et 14 définissent un plan moyen Pm coupant axialement les deux parties rigides 20 et 22.

Ainsi, la pièce de liaison, dont les premières portions de fixation 20' et 22' des parties rigides 20 et 22 sont en appui fixe, est apte à fléchir élastiquement selon une direction Dp perpendiculaire au plan moyen Pm sous l'effet d'une force F exercée sur chacune des branches 12 et 14. En effet, compte tenu de la section des branches 12 et 14 par rapport à la section des parties rigides 20 et 22, le rayon de courbure du fléchissement de la pièce de liaison est minimum au centre de la partie flexible 10.

La somme des forces F exercées sur les branches perpendiculairement au plan moyen Pm, apte à fléchir la pièce de liaison sans la rompre, ne permettrait pas de fléchir la pièce de liaison selon une direction perpendiculaire à l'axe A de la pièce et contenue dans le plan moyen Pm. En effet, dans ce cas une des branches travaillerait en extension et l'autre en compression, ce qui bloquerait la flexion. Ainsi, on obtient une direction de flexion privilégiée, ce qui, comme on l'expliquera plus en détails dans la suite de la description, présente un avantage dans le cadre de l'application envisagée.

Afin d'obtenir une amplitude de flexion optimale par rapport aux dimensions de la pièce de liaison et une direction privilégiée de fléchissement, les deux branches 12 et 14 sont séparées d'une distance au moins égale à la longueur de leur portion parallèle. On comprend bien que plus les branches 12, 14 sont rapprochées, plus la flexion pourra se faire selon un axe non strictement perpendiculaire au plan moyen Pm et que la longueur totale des branches 12 et 14 diminuant, l'effort nécessaire à ladite flexion devra être plus important. Toutefois, l'espacement entre les deux branches 12 et 14 est limité par l'espace disponible pour insérer ladite pièce, aussi bien transversalement que selon l'axe longitudinal, et par conséquent on prévoit que la distance qui sépare les extrémités desdites branches reliées deux à deux est comprise entre 1,5 et 2,5 fois la distance qui sépare lesdites branches.

Afin de comparer la contrainte subie par une pièce de liaison conforme à l'invention et la contrainte subie par une tige de liaison simple de l'art antérieur on réalise une tige de diamètre 1 dont on souhaite augmenter l'amplitude de flexion de 50%. Pour ce faire, le diamètre de la tige de liaison simple doit être diminué de 10%, ce qui a pour conséquence d'augmenter de 35% la contrainte qu'elle subit. En revanche, pour obtenir la même amplitude de flexion, la pièce de liaison conforme à l'invention comporte des branches dont le diamètre doit représenter 75% du diamètre initial de la tige de diamètre 1, tandis que la contrainte subie par les branches n'augmente que de 13%.

Ainsi, les résultats de ces mesures montrent que la pièce de liaison conforme à l'invention subit moins de contraintes qu'une tige de liaison classique pour une même flexion ce qui entraîne une moindre fatigue de la pièce de liaison et donc une durée de vie plus importante que celle des tiges de l'art antérieur. En outre, la pièce conserve d'autant plus ses propriétés élastiques que les contraintes qu'elle subit sont moindres.

On se référera maintenant à la Figure 2 pour décrire la déformation de la pièce de liaison par rapport aux mouvements relatifs des éléments d'ancrage 26 et 28 illustrés.

On retrouve sur la Figure 2 la pièce de liaison reliant les deux éléments d'ancrage 26 et 28 par ses deux parties rigides 20 et 22. Les deux éléments d'ancrage 26 et 28 sont parallèles entre eux et traversés par un plan axial commun Pa. La pièce de liaison est fixée sur les éléments d'ancrage 26 et 28 de façon que le plan moyen Pm défini par les branches 12 et 14 soit sensiblement perpendiculaire au plan axial commun Pa.

Les éléments d'ancrage 26 et 28 présentent une tige filetée 30 surmontée d'une tête 32 formant un U dont la paroi interne est filetée de façon qu'un élément 34 formant vis s'y adapte. Ainsi, les premières portions de fixation 20' et 22' des parties rigides 20 et 22 sont encastrées dans les têtes 32 des éléments d'ancrage 26 et 28 respectivement et sont bloqués sur ces derniers par serrage des éléments 34 formant vis.

De la sorte, lorsque les tiges filetées 30 des éléments d'ancrage tendent à se rapprocher sous la contrainte des forces opposées T et -T, contenues dans le plan Pa et sensiblement parallèles à l'axe A, les éléments d'ancrage 26 et 28 déforment la pièce de liaison qui forme un arc compris dans le plan Pa. La contrainte induit une flexion de la pièce de liaison au niveau de ses branches 12 et 14 perpendiculairement au plan moyen Pm. Lorsque la contrainte cesse, la pièce de liaison retrouve sa forme primitive rectiligne et les tiges filetées des éléments d'ancrage 30 retrouvent leur position relative.

Le mécanisme de flexion élastique de la pièce de liaison et des éléments d'ancrage décrit ci-dessus est le même lorsque les tiges filetées 30 des éléments d'ancrage 26 et 28 sont respectivement éloignées l'une de l'autre, la pièce de liaison formant un arc inverse.

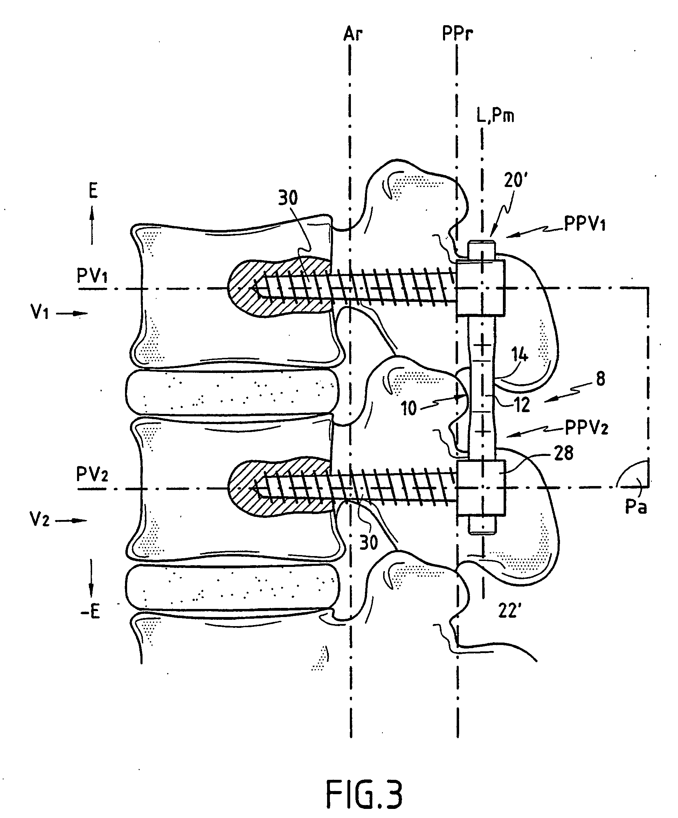

On se référera maintenant à la Figure 3 pour d'écrire l'utilisation de la pièce de liaison 8 dans un système de stabilisation vertébrale destiné à solidariser au moins deux vertèbres V1 et V2.

Les vertèbres V1 et V2 présentent chacune un plan moyen PV1 et PV2 sensiblement perpendiculaire à l'axe Ar du rachis qu'elles forment et une paroi postérieure PPV1 et PPV2 définissant un plan moyen postérieur PPr dudit rachis.

Le système de stabilisation comporte au moins deux éléments d'ancrage 26 et 28 vissés chacun dans la paroi postérieure PPV1 et PPV2 des vertèbres V1 et V2, respectivement, de façon que la ligne L qui coupe les deux éléments d'ancrage 26 et 28 soit sensiblement parallèle audit axe Ar du rachis. Une pièce de liaison 8 relie les deux éléments d'ancrage 26 et 28 par ses deux premières portions de fixation 20' et 22' de façon que ledit plan moyen Pm défini par lesdites deux branches 12 et 14 soit sensiblement parallèle audit plan moyen postérieur dudit rachis PPr. De la sorte, les vertèbres V1 et V2, reliées dans leur partie postérieure, présentent une mobilité relative selon l'axe Ar du rachis.

Ainsi, lorsque le rachis est mis en extension, les vertèbres V1 et V2 tendent à s'écarter l'une de l'autre selon E et -E respectivement, ce qui entraîne l'écartement des tiges filetées 30 l'une de l'autre également, induisant la déformation de la pièce de liaison 8, et particulièrement de sa partie flexible 10. La pièce de liaison ainsi déformée forme une concavité opposée au rachis.

Lorsque le rachis est mis en flexion, l'effet inverse se produit et les vertèbres V1 et V2 tendent à se rapprocher, ce qui induit la déformation de la pièce de liaison de façon à former une concavité dirigée vers le rachis.

On comprend que la pièce de liaison 8 conforme à l'invention procure une plus grande mobilité des vertèbres les unes par rapport aux autres puisque qu'elle présente une amplitude de flexion plus importante que les tiges de liaison de l'art antérieur. Par ailleurs, du fait de sa construction, avec deux branches parallèles 12, 14 réunies dans leurs extrémités et prolongées par les parties rigides 20, 22, la pièce de liaison 8 permet la flexion et l'extension du rachis dans le plan Pa tout en limitant le fléchissement dans le plan Pm perpendiculaire à Pa. Ainsi, le rachis est stabilisé latéralement et le déplacement relatif des vertèbres est limité.

Selon un mode particulier de réalisation, non représenté, la pièce de liaison comprend trois parties rigides, formant tiges reliées entre elles par une partie flexible. Pour ce faire, la partie rigide centrale comprend deux deuxièmes portions prolongeant de chaque côté ladite portion de fixation, lesdites deuxièmes portions prolongeant respectivement les deux extrémités de deux branches reliées entre elles, des deux parties flexibles. Les parties rigides prolongent chacune des extrémités des branches reliées entre elles de façon que les plans moyens des parties flexibles soient sensiblement situés dans un même plan. Ainsi, on obtient une pièce longitudinale présentant deux premières parties rigides à chacune de ses extrémités et une partie rigide centrale entre les deux premières, les parties rigides étant reliées deux à deux par des parties flexibles.

Ainsi, la pièce de liaison maintient un espacement entre trois éléments d'ancrage qu'elle relie, ces derniers étant fixés dans trois vertèbres sensiblement équidistantes, de façon à constituer un alignement. Chaque partie rigide de la pièce de liaison est fixée à un élément d'ancrage de façon à obtenir une partie flexible entre les vertèbres prises deux à deux. De la sorte, trois vertèbres sont stabilisées avec une seule pièce de liaison unique, ce qui permet de diminuer le temps de montage de l'ensemble du système de stabilisation et par conséquent le temps opératoire. En outre, les trois vertèbres étant reliées par une pièce unique de liaison leur mobilité relative, les unes par rapport aux autres, est mieux contrôlée.

Il va de soi qu'on ne sortirait pas du cadre de l'invention en prévoyant des pièces de liaison comportant plus de trois parties rigides séparées par des parties flexibles.

## Revendications

1. Pièce de liaison destinée à maintenir un espacement entre au moins deux éléments d'ancrage vissés dans des vertèbres, comprenant au moins :
- une partie flexible divisée en deux branches continues espacées l'une de l'autre, lesdites branches étant sensiblement symétriques par rapport à l'axe longitudinal de ladite pièce, les extrémités desdites branches étant reliées entre elles deux à deux et définissant un premier plan moyen, et,
- deux parties rigides formant tiges, présentant une première portion de fixation et une deuxième portion, chaque dite deuxième portion desdites deux parties rigides prolongeant respectivement dans des directions opposées lesdites extrémités desdites branches reliées entre elles deux à deux, la section droite de chacune desdites branches étant inférieure à la section droite desdites parties rigides de façon que ladite pièce de liaison, dont lesdites portions de fixation sont fixées respectivement sur chacun des deux éléments d'ancrage, soit apte à fléchir élastiquement autour d'un axe perpendiculaire à l'axe longitudinal, ledit axe perpendiculaire étant contenu dans ledit plan moyen de la pièce de liaison lors du déplacement relatif des vertèbres, par quoi les vertèbres, maintenues espacées l'une de l'autre, sont mobiles l'une par rapport à l'autre.

2. Pièce de liaison selon la revendication 1, **caractérisée en ce qu'**elle comprend n parties rigides entre lesquelles sont interposées n-1 parties flexibles selon l'axe longitudinal de ladite pièce, chaque partie rigide située entre deux parties flexibles comportant une première portion de fixation et deux deuxièmes portions, chacune desdites deuxièmes portions étant située de chaque côté de ladite première portion de fixation, lesdites deuxièmes portions prolongeant respectivement les extrémités des branches desdites deux parties flexibles de façon que les plans moyens de toutes les parties flexibles soient sensiblement situés dans un même plan, et **en ce que** les parties rigides situées aux deux extrémités de ladite pièce présente une seule deuxième portion prolongeant les extrémités des branches des parties flexibles par quoi ladite pièce est apte à relier n éléments d'ancrage.

3. Pièce de liaison selon la revendication 1 ou 2, **caractérisée en ce que** la somme de la surface des sections desdites deux branches est inférieure à la surface de la section desdites parties rigides formant tiges.

4. Pièce de liaison selon l'une quelconque des revendications 1 à 3, **caractérisée en ce que** lesdites branches présentent des portions parallèles entre elles et à l'axe longitudinal de ladite pièce et **en ce que** la distance qui sépare lesdites branches est au moins égale à la longueur desdites portions.

5. Pièce de liaison selon la revendication 4, **caractérisée en ce que** la distance qui sépare les extrémités desdites branches reliées deux à deux est comprise entre 1,5 et 2,5 fois la distance qui sépare lesdites branches.

6. Pièce de liaison selon l'une quelconque des revendications 1 à 5, **caractérisée en ce que** la section desdites parties rigides formant tiges est circulaire.

7. Pièce de liaison selon l'une quelconque des revendications 1 à 6, **caractérisée en ce que** le matériau dans lequel est réalisée ladite pièce est un alliage de titane.

8. Système de stabilisation vertébrale destiné à solidariser au moins deux vertèbres, lesdites vertèbres présentant chacune un plan moyen sensiblement perpendiculaire à l'axe du rachis qu'elles forment et une paroi postérieure définissant un plan moyen postérieur dudit rachis, ledit système comportant au moins deux éléments d'ancrage aptes à être fixés chacun dans la paroi postérieure d'une vertèbre de façon que la ligne, qui coupe lesdits deux éléments d'ancrage, soit sensiblement parallèle audit axe du rachis,
**caractérisé en ce qu'**il comprend au moins une pièce de liaison selon l'une quelconque des revendications 1 à 7 apte à relier lesdits deux éléments d'ancrage par lesdites deux parties rigides de façon que ledit plan moyen défini par lesdites deux branches soit sensiblement parallèle audit plan moyen postérieur dudit rachis, par quoi lesdites vertèbres, reliées dans leur partie postérieure, présentent une mobilité relative selon ledit axe dudit rachis.

## Patentansprüche

1. Verbindungsstück zum Halten eines Abstands zwischen wenigstens zwei in Wirbeln verschraubten Verankerungselementen, das wenigstens aufweist:
- ein bewegliches Teil, das in zwei fortlaufende voneinander beabstandete Äste getrennt ist, wobei die Äste im Wesentlichen symmetrisch bezüglich der Längsachse des Stücks verlaufen, wobei die Enden der Äste paarweise untereinander verbunden sind und eine erste mittlere Ebene definieren, und
- zwei Stangen bildende steife Teile, die einen ersten Befestigungsabschnitt und einen zweiten Abschnitt aufweisen, wobei jeder genannte zweite Abschnitt der beiden steifen Teile jeweils die Enden der untereinander paarweise verbundenen Äste in entgegengesetzte Richtungen verlängert, wobei der Querschnitt von jedem der Äste kleiner ist als der Querschnitt der steifen Teile, derart dass das Verbindungsstück, dessen Befestigungsabschnitte jeweils auf jedem der beiden Verankerungselemente befestigt sind, sich elastisch um eine Achse senkrecht zur Längsachse biegen kann, wobei die senkrechte Achse in der mittleren Ebene des Verbindungsstücks bei der jeweiligen Bewegung der Wirbel enthalten ist, wodurch die voneinander beabstandeten Wirbel, jeweils gegeneinander beweglich sind.

2. Verbindungsstück nach Anspruch 1, **dadurch gekennzeichnet, dass** es n steife Teile aufweist, zwischen denen n-1 bewegliche Teile entlang der Längsachse des Stücks eingefügt sind, wobei jedes zwischen zwei beweglichen Teilen liegende steife Stück einen ersten Befestigungsabschnitt und zwei zweite Abschnitte aufweist, wobei jeder der zweiten Abschnitte auf jeder Seite des ersten Befestigungsabschnitts liegt, wobei die zweiten Abschnitte jeweils die Enden der Äste der beiden beweglichen Teile verlängern, derart, dass die mittleren Ebenen aller beweglichen Teile im Wesentlichen in der selben Ebene liegen, und dass die an den beiden Enden des Stücks liegenden steifen Teile einen einzigen zweiten Abschnitt aufweisen, der die Enden der Äste der beweglichen Teile verlängert, wodurch das Stück n Verankerungselemente verbinden kann.

3. Verbindungsstück nach Anspruch 1 oder 2, **dadurch gekennzeichnet, dass** die Summe der Fläche der Querschnitte der beiden Äste kleiner ist als die Fläche des Querschnitts der Stangen bildenden steifen Teile.

4. Verbindungsstück nach einem der Ansprüche 1 bis 3, **dadurch gekennzeichnet, dass** die Äste Abschnitte aufweisen, die parallel zueinander und zur Längsachse des Stücks verlaufen, und dass der Abstand, der die Äste trennt, wenigstens gleich der Länge der Abschnitte ist.

5. Verbindungsstück nach Anspruch 4, **dadurch gekennzeichnet, dass** der Abstand, der die Enden der paarweise verbundenen Äste trennt, zwischen dem 1,5- und 2,5-fachen Abstand, der die Äste trennt, liegt.

6. Verbindungsstück nach einem der Ansprüche 1 bis 5, **dadurch gekennzeichnet, dass** der Querschnitt der die Stangen bildenden steifen Teile kreisförmig ist.

7. Verbindungsstück nach einem der Ansprüche 1 bis 6, **dadurch gekennzeichnet, dass** das Material, aus dem das Stück hergestellt ist, eine Titanlegierung ist.

8. System zur vertebralen Stabilisierung, das zum Verbinden von wenigstens zwei Wirbeln bestimmt ist, wobei die Wirbel jeweils eine mittlere Ebene, die im Wesentlichen senkrecht zur Achse der Wirbelsäule, die sie bilden, verläuft, und eine Hinterwand aufweisen, die eine hintere mittlere Ebene der Wirbelsäule definieren, wobei das System wenigstens zwei Verankerungselemente aufweist, die jeweils in der Hinterwand eines Wirbels befestigt werden können, derart, dass die Linie, die die beiden Verankerungselemente schneidet, im Wesentlichen parallel zu der Achse der Wirbelsäule verläuft,
**dadurch gekennzeichnet, dass** es wenigstens ein Verbindungsstück nach einem der Ansprüche 1 bis 7 aufweist, das die beiden Verankerungselemente durch die beiden steifen Stücke verbinden kann, derart, dass die durch die beiden Äste definierte mittlere Ebene im Wesentlichen parallel zur hinteren mittleren Ebene der Wirbelsäule verläuft, wodurch die Wirbel, die in ihrem Hinterteil verbunden sein, eine Beweglichkeit jeweils entlang der Achse der Wirbelsäule aufweisen.

## Claims

1. A connecting member for maintaining the spacing between at least two anchor members screwed into vertebrae, comprising at least:
- a flexible part divided into two spaced continuous branches that are substantially symmetrical about a longitudinal axis of said member, the ends of said branches being interconnected in pairs and defining a first median plane, and
- two rigid rod-forming parts each having a fixing first portion and a second portion, each of said second portions of said two rigid parts respectively extending said ends of said branches interconnected in pairs in opposite directions, the cross-section of each of said branches being less than the cross-section of said rigid parts so that said connecting member, said fixing portions of which are fixed to respective anchor members, is able to bend elastically about an axis that is perpendicular to the longitudinal axis, said perpendicular axis being contained in said median plane of the connecting member on relative movement of the vertebrae, whereby the vertebrae, which are held spaced relative to each other, are movable relative to each other.

2. A connecting member according to claim 1, **characterized in that** it comprises n rigid parts between which there are disposed n-1 flexible parts along the longitudinal axis of said member, each rigid part situated between two flexible parts having a first fixing portion and two second portions, each of said second portions being situated at a respective end of said first fixing portion, said second portions extending respective ends of the branches of said two flexible parts so that the median planes of all the flexible parts are substantially the same, and **in that** the rigid parts situated at both ends of said member advantageously have respective single second portions extending the ends of the branches of the flexible parts whereby said member is adapted to interconnect n anchor members.

3. A connecting member according to claim 1 or 2, **characterized in that** the sum of the surface areas of the sections of said two branches is less than the surface area of the section of said rigid rod-forming parts.

4. A connecting member according to any one of claims 1 to 3, **characterized in that** said branches have portions parallel to each other and to the longitudinal axis of said member and **in that** the distance between said branches is at least equal to the length of said portions.

5. A connecting member according to claim 4, **characterized in that** the distance between the ends of said branches connected in pairs lies in the range 1.5 times to 2.5 times the distance between said branches.

6. A connecting member according to any one of claims 1 to 5, **characterized in that** the section of said rigid rod-forming parts is circular.

7. A connecting member according to any one of claims 1 to 6, **characterized in that** the material from which is made said member is titanium alloy.

8. A vertebral stabilization system for fastening together at least two vertebrae each having a median plane substantially perpendicular to the axis of the spine of which they are part and a posterior wall defining a posterior median plane of said spine, said system comprising at least two anchor members each adapted to be fixed into the posterior wall of a vertebra so that a line which intersects said two anchor members is substantially parallel to said axis of the spine,
the system being **characterized in that** it comprises at least one connecting member according to any one of claims 1 to 7 whose two rigid parts are adapted to interconnect said two anchor members so that said median plane defined by said two branches is substantially parallel to said posterior median plane of said spine, whereby said vertebrae, which are interconnected in their posterior portions, are relatively movable along said axis of said spine.
